# EUROPEAN PATENT SPECIFICATION

(11) **EP 3 787 791 B1**
(45) Date of publication and mention of the grant of the patent: **15.12.2021**
(21) Application number: 19726303.1
(22) Date of filing: 30.04.2019
(51) Int. Cl.: B01L 3/00, B29C 65/50, B29C 65/00, G01N 35/04, G01N 35/02, B29C 65/78, B65B 7/16, B65B 51/14

(54) **SEALER OF GAS-TIGHT SEALING OF WELLS IN PCR PLATES BY THIS SEALER**
ABDICHTUNGSMITTEL ZUM GASDICHTEN ABDICHTEN VON VERTIEFUNGEN IN PCR-PLATTEN DURCH DIESES ABDICHTUNGSMITTEL
DISPOSITIF DE SCELLEMENT ÉTANCHE AUX GAZ DE PUITS DANS DES PLAQUES DE PCR AU MOYEN DE CE DISPOSITIF DE SCELLEMENT

(30) Priority: 02.05.2018 CZ 201800207 U
(43) Date of publication of application: 10.03.2021
(73) Proprietor: GENEPROOF A.S., 61900 Brno, Dolni Herspice (CZ)
(72) Inventor: DENDIS, Milos, 67906 Jedovnice (CZ); HORVATH, Radek, 667 01 Zidlochovice (CZ); PETRIK, Martin, 25169 Velke Popovice (CZ); STARY, Tomas, 39301 Pelhrimov (CZ); PRIDAL, Zdenek, 77900 Olomouc, Lazce (CZ); KNOPF, Petr, 78501 Sternberk (CZ); SVARC, Marek, 75125 Vykleky (CZ)
(74) Representative: Dadej, Leopold
(86) International application number: PCT/CZ2019/050018
(87) International publication number: WO 2019/210890

(56) References cited:
- WO-A1-2015/192330
- GB-A- 364 397
- US-A- 1 808 602
- US-A1- 2002 021 986
- US-A1- 2013 230 860
- US-B1- 6 408 595

## Description

### Field of the Invention

The invention relates to analysers for automatic diagnostic PCR process, and particularly to a sealer for sealing wells in PCR plates.

### Background of the Invention

PCR analysis (Polymerase Chain Reaction), which is based on the amplification of nucleic acid segments by polymerase chain reaction, is a known diagnostic method for identifying biological material. Part of the diagnostic PCR process is the dosing of individual solutions for PCR analysis into tubes often arranged in a PCR plate manufactured in standardized dimensions. Due to the thermal processes during analysis, this PCR plate must be sealed, which is usually done by using a sealing film of corresponding dimensions made for this purpose. These rectangular-shaped sealing film sheets are standardly attached to the PCR plate manually, most often by pressing with hands, spatula or hand roller. During the following analysis, the PCR plate with the sealing film is further covered by a pressure lid, which, however, does not have a sealing function and usually performs the upper heating function so that the solution is heated uniformly, i.e. not only from the bottom, thereby preventing condensation of the solution in the upper part of the PCR plate or on the surface of the sealing film. The diagnostic PCR process consists of a sequence of consecutive events and operations lasting several hours, approximately 2 to 4 hours. In the first step, nucleic acids are isolated from a clinical material, in the second step, the so called PCR setup is performed - preparing the actual PCR reactions in the PCR plate and adding the samples to specific positions in the PCR plate, and in the third step, the actual PCR reaction is performed, wherein the operation of manual sealing of the sealing film for the PCR plate is performed after about 1 to 2 hours of the diagnostic PCR process, specifically after the first two steps before the actual PCR reaction is initiated. The problem of manual sealing in laboratory analysers is that after batching the solutions into a PCR plate, the process must be discontinued, the analyser for the automated diagnostic PCR process must be opened, or the PCR plate must be ejected/withdrawn and provided with the sealing film by sealing, for example, by hands of a laboratory technician. The PCR plate provided with the sealing film is subsequently put/inserted back into the analyser, the analyser is sealed and proceeds with further automatic analysis steps. Such a procedure is disadvantageous because, thanks to this single manual intervention into an otherwise fully automated process, it is not possible to leave the analyser unattended for the entire analysis period, it is necessary to monitor the appropriate time to attach the sealing film to the PCR plate, while the likelihood of an error caused by the human factor increases, such as possible improper attachment of the sealing film, the contamination of the solutions or spillage thereof, as well as untimely execution of this manual operation. The whole process thus becomes only semi-automatic.

Sealing film specially manufactured for the purpose of chemical analyses is commercially available either in large rolls for various larger industrial analytical machines, or, as in the case of PCR analyses, directly in rectangular sheets of standard dimensions corresponding to conventional PCR plate dimensions where the sheets are rectangular in shape, with longer lateral sides and further with shorter sides of the rectangle forming the first and second ends of the sealing film. Similar to stickers, these commercially produced sheets can be provided with a lid/carrier layer that needs to be removed manually prior to use.

In the large-volume scale of industrially performed analyses other than PCR, sealing of analytical plates is fully automated using rolls with wound sealing film. Laboratory (bench top) automated or semi-automatic analytical plate sealers that work with sealing film wound in a reusable container are available on the market, they can also use the heat seal function and by their design are not suitable for placing into an automatic diagnostic process for PCR analysis or into liquid samples handling devices. They do not allow sealing of PCR plates directly in smaller automated assemblies. It is therefore not possible to incorporate these industrially manufactured sealing film discs into smaller laboratory analysers for automatic diagnostic PCR process, and adjusting the analyser to use these rolls would necessarily result in spatial magnification of the analyser or costly adaptation of the standard dimensions of these rolls to smaller laboratory analysers, and predominantly to a necessary development of the unwinding and pressing device installed in the analyser design, wherein a number of other related problems would also need to be addressed. For the above reasons, such laboratory equipment has not yet been designed.

Patent document US2008115885 uses a manually operated iron to heat seal the sealing medium to the sample plate.

Patent document US2012196774 describes a general sealing solution using rigid materials with modified surface, such as a film of a suitable solid material, a pressure sensitive adhesive pressed down to a sealed surface using pressure, for example using a movable platform, rolling strip, roller.

Also known on the market is the automatic PCR plate sealer, hereinafter referred to as the sealer ("plate sealer"), which is used for gas-tight sealing of the wells of PCR plates or other analytical plates by sealing films attached to the sealed surface by pressure, for example by means of a movable platform, rolling strip, roller, and the like. This sealer is suitable for liquid sample handling systems and possibly suitable for the use in automatic diagnostic processes. The analytical microtiter plate is first placed into the sealer outside the analytical instrument or sealed manually and, after sealing the sealing film using, for example, a roll or an iron with thermal sealing, it is removed and transferred to the target position in the analysis apparatus. It is not possible to implement this sealer into an analyser for automatic diagnostic PCR process, or it would be costly.

An alternative solution is to use a top pressure plate in the so-called bench top sealers, which is often temperable for plate sealing purposes by thermofoils. Tempering, however, can lead to impairment of the plate contents, so the temperature must be optimized to avoid deterioration of the plate content while being sufficient to seal the sealing film.

Patent document US6917035 discloses an automated press device plate in the form of a planar plate for sealing the microtiter plates with a typically 0.5-2 mm thick film or flexible elastically mouldable rubber/silicone or thermoplastic elastomer mattress, wherein the press device is slidable in a vertical direction.

Patent document US2005074360 discloses a microtiter plate sealer for biological samples using a sealing film with a flat pressure head, wherein the sealing film can be unwound automatically from the stock disc and guided using guide rollers to the microtiter plate surface, wherein separation of the sealing film carrier layer from the actual sealing film occurs before entering the microtiter plate. Subsequently, the sealed plates are stacked for further use.

Patent document US6408595 discloses a stand-alone microtiter plate sealer fixed by suction cups to a solid surface, with a stable base with side panels between which an insertion panel with a microtiter plate is loosely placed in rails, and a sealing cover loosely laid thereon, which is retracted in contact with a rotating roller operated manually by a bidirectional handle. Similarly, patent document US6543203 discloses a sealer in the form of a box with a manual crank into which an analytical plate is placed with a cover thereon, where in one direction of rotation the cover of the analytical plate can be sealed to the analytical plate and separated in the opposite direction of rotation.

Patent document EP2431747 discloses a rotatable sealing pressure roller for pressing a sealing film placed on the top surface of a microtiter plate, located at the inlet/outlet of an automated liquid samples handling space, which also serves as a movable mechanism for inserting or withdrawing the microtiter plate from/to the said space. Alternatively, the microtiter plate may be shifted and pushed between two such rollers, where one of the rollers pushes from the bottom on the passing microtiter plate and the other from the top on the sealing film.

Other plate sealers with rollers are disclosed in patent documents US2002/021986A1, US2013/230860A1, WO2015/192330A1.

Another alternative method is, for example, to use adhesive aluminium film, wherein there is a risk of contamination of the contents of the plate wells with the adhesive.

In the case of automatic diagnostic lines, the problem of sealing the PCR plates is solved by:
- Using specific plastic tubes instead of PCR plates and sealing with lids instead of the sealing film.
- Manual transfer of the sealing film and sealing on the PCR plate by hand. The diagnostic process is interrupted; the operator manually seals the PCR plate with the sealing film and transfers it to the PCR apparatus.
- Transferring the sealing film to the PCR plate using suction cups and pressing in the cycler with the lid, while undesirable film displacement, exposing the outer wells, vaporizing the contents of the well and hazardous contamination of the analyser environment can occur.

Patent document US20120058516 refers to sealing of an analytical PCR plate by heating the sealing film with the aid of a sealing lid and the possibility of automation.

Patent document US80030080 discloses sealing of PCR plates using a flexible barrier (thermoplastic elastomer) or a heated cycler lid with flexible sealing by heating and pressure. Furthermore, a robotic arm or other manipulation device is used to release the sealing pressure, to lift the lid vertically and transfer it to a position outside the analytical plate.

### Summary of the Invention

The above mentioned drawbacks are solved by a sealer for gas-tight sealing of wells in PCR plates using a sealing film, characterized in that the sealer comprises a hinge and further a sealer body in the form of a longitudinally cut-out cylinder comprising a cylindrical shell area for forcefully transferring the sealing film to the upper rigid edge part of the PCR plate wells by pressure and swaying-rolling motion of the sealer body, and a cut-out shell area, wherein the sealer body is rotatably connected to the hinge by means of a joint with a rotation axis coincident with a central axis of the cylindrical surface and the hinge is further slidably connected to the positioning device for horizontal and/or vertical movement of the sealer in a plane perpendicular to the axis of rotation of the sealer body; the sealer further comprises at least one clamping element for clamping both ends of the sealing film to the sealer body. The hinge is further connected to a positioning device for horizontal and/or vertical movement of the sealer in a plane perpendicular to the axis of rotation of the sealer body. The clamping element is in the form of a housing, where the lower part of the housing dimensionally and shape-wise copies the cut-out shell area of ferromagnetic material and there is at least one magnet inside the lower part of the housing, and the housing is further connected to the cut-out shell area by means of a barrier consisting of a body provided at its ends with two heads, where one barrier head is housed in the hollow body of the sealer and the second barrier head is housed in the housing, and the barrier body extends through an opening in the cut-out shell area and an opening in the lower housing part, where each opening is smaller than the respective head, and the barrier body is provided with a spreader spring that overcomes the magnetic force of the sealer body separated from the housing but does not overcome the magnetic force of the housing pressed to the sealer body.

The described sealer is suitable for gas-tight sealing of wells in PCR plates for automatic diagnostic PCR process, in which a PCR plate at least partially filled with chemicals is gas-tight sealed by a sealing film, which is transferred and pressed to an upper rigid edge portion of the PCR plate wells, characterized in that first, before the diagnostic PCR process is initiated in an analyser, a sealing film is placed on a sealer body and is attached to the sealer body, and the sealer is set in its parking position so that it does not obstruct, further, during the diagnostic PCR process after the PCR plate wells are filled with chemicals, the sealer is guided through the positioning device to the gas-tight sealing position and gas-tight sealing using the sealing film is performed, in which the sealing film is pressed and transferred from the sealer body provided with the sealing film to the upper rigid edge portion of the PCR plate by pressure and swaying-rolling motion of the sealer body, wherein after sealing by the sealing film is performed, the sealer is guided back to the parking position. Subsequently, the polymerase chain reaction process, the so-called PCR analysis, is initiated.

The main advantage and problem being solved of the above described sealer for gas-tight sealing of PCR plates is that it can be used in relatively small fully automated, or semi-automated analysers for an automatic diagnostic PCR process, where automation of gas-tight sealing of PCR plates allows full automation of laboratory or mobile analysers for automatic diagnostic PCR process, facilitates operator work, the presence of whom is now required only prior to initiating and after finishing a several-hour long analysis, thereby limiting error rate caused by human factor and ensuring process sterility, as well as reducing the risk of staining the operator with chemicals.

Essential for the automation of the diagnostic PCR process is that the sealing film is prepared prior to initiation of the analysis on the sealer body, not laid directly on the PCR plate prior to the analysis or prior to pressure sealing. This allows access to the wells for automatic chemical batching, and subsequently, sealing of the films can be carried out automatically without the need to manually lay the film on the PCR plates during the process.

By gas-tight sealing of the wells in PCR plates, it is meant to provide a sufficiently strong bond between the inlet upper edge of the well hole in the PCR plate by pressure acting on the rigid upper edge through a sealing film specially commercially manufactured for this purpose or similar sealing film, which has the ability to form coherent physical interactions with the surface of the PCR plate upon application of a certain action of force. The joint must be strong enough to withstand the pressure exerted by the chemicals during heating. Thus, the need for bond strength will depend, for example, on the chemicals used, the temperature and the heating time.

The gas-tight or eventually liquid-tight sealing of the wells in PCR plates is purposeful only when these containers contain chemicals. These include, for example, reagents for PCR analysis, liquid samples or stock inlet solutions, and the like, which can be filled into these containers, for example, by pipetting, wherein they are usually not completely filled with chemicals, but only partially. It is also possible to fill only some of the wells of a PCR plate with chemicals.

The invention has been developed namely for conventionally used PCR plates used for PCR automatic diagnostic PCR, but can also be used for other analytical plates, such as microtiter plates for analysis and manipulation of multiple liquid samples.

The automated process means automated or semi-automated determination of properties or composition of liquid samples in the analyser for diagnostic PCR process by means of programmed steps, with minimization of operator intervention, in particular, the steps of activating the sealer mechanism and positioning, pressing or further manipulating with the sealer body, as well as other operations concerning, for example, chemical pipetting, heating, etc. are automated in the analyser for automatic diagnostic PCR process.

Sealer is a device that performs gas-tight/vapour-tight sealing of originally open space of wells in a PCR plate placed in a separate stand or directly in an analyser for automatic diagnostic PCR process. This sealing is effected by a sealing film, by means of physical retaining forces, using pressure.

In a preferred embodiment, the cylindrical surface of the shell occupies a cylinder shell in the range of 90° to 270° angle of the circular base, preferably 180°, and the remaining portion up to 360° is formed by the cut-out shell area. In a preferred embodiment, the sealer body is semi-cylindrical in shape.

The cut-out shell area can be made as a typical circular segment of the circular base or as a straight line between the end points of the missing part of the circle. In a preferred embodiment, a flat cut-out shell area is provided for clamping the ends of the sealing film, where the flat surface ensures contact between the sealer body and the clamping element and thus allows a simpler design of the clamping element.

In a preferred embodiment, the attachment of the sealing film to the semi-cylindrical sealer body is accomplished by manually inserting the first and second ends of the sealing film between the straight cut-out shell area of the semi-cylindrical sealer body and at least one clamping element with subsequent pressing of the clamping element to the sealer body, and gas-tight sealing is accomplished by means of a sealing film by swaying-rolling motion of the sealer body. Prior to starting the next insertion of the sealing film, the clamping element is separated from the sealer body.

The semi-cylindrical sealer body is a specially formed sealer element that allows swaying-rolling motion, for example, over the surface of PCR plates. Preferably, the dimensions of the sealer body are adapted to the dimensions of the PCR plates so that the entire upper surface of the wells and their rigid edge portions on the PCR plate are covered by the swaying and rolling motion of the sealer body, and the sealing film can be thus pressed to the PCR plate in its entirety by at least forward swaying motion. Depending on the developed force, it is possible to adjust the sealing films so that several forward and backward movements are made. Practically, it depends on the force developed; it is possible to adjust the sealing film so that multiple forward back and forth motions will be carried out.

One dimension of the rectangular sheet of the sealing film does not have to be exactly the same as the height of the semi-cylinder, although it is appropriate, and the second dimension must be matching so that the sealing film can be attached to the sealer body and the sealing film must also overlay the space on the PCR plate required for sealing, i.e. determined by wells and the rigid edge portion thereof, so that the sealing film can be clamped to the upper rigid edge portion of the PCR plate wells. The top and bottom surfaces of the sealing film are adapted so that the bottom surface, upon exertion of pressure on the sealing film, forms adherent physical interactions with the PCR plate, and at the same time the top surface of the sealing film does not adhere to the sealer body.

In a preferred embodiment, the cylindrical shell area has a rectangular shape with minimum dimensions covering the area defined by the wells and the rigid edge thereof on the PCR plate to be sealed.

The clamping element may be any means ensuring retaining the sealing film on the sealer body. These may be magnets attracting each other, where one piece is implemented into the sealer body and the other is adjacent to it through a sealing film or magnets placed in the housing, wherein one of the magnetic counterparts may be replaced by a ferromagnetic material, but it may also be various clips, pins and other attachment elements.

In a preferred embodiment, there are four magnets placed in the vicinity of the corners of the rectangular lower part of the housing and the housing is connected with the cut-out shell area by four barriers provided with springs. For example, the barrier may be a metal rod with threads in the end portions and a metal nut at each end; the housing may be, for example, plastic. The advantage of using this clamping element is easy handling and eventual cleaning.

When the housing is separated from the sealer body, it is kept separate by means of the spring, until it is pressed to the sealer body. After compression, the housing retains by the sealer body by virtue of greater magnetic force, while at the same time, if the sealing film has been clamped between them before, it holds the sealing film by the ends thereof, until the sealing film is transferred and pressed to the PCR plate, where the forces between the sealing film and the PCR plate prevail, and by moving the sealer, the ends of the sealing film initially fixed by the magnetic force between the clamping element and the sealer body are drawn out.

Before starting the next insertion of the sealing film, the clamping element is separated from the cut-out shell area, and thus separated, it is then held by means of the above-mentioned barriers with springs.

In a preferred embodiment, the cut-out shell area of the sealer body comprises at least one resilient pin with a tip of electrical current conducting material and connected to the power supply, where the tip provides greater friction and prevents movement of the attached sealing film, and the power supply is used to detect the location of the sealing film. If current passes through the resilient pin, that is, if the pin directly touches the pressure element, it is detected that the sealing film is not positioned between the pressure element and the sealer body. On the contrary, if current does not pass through the resilient pin, it is detected that the sealing film is positioned correctly. Preferably, there are four pins. Most preferably, the pins are located near the edges of the end portions of the inserted attached sealing film.

In a preferred embodiment, the surface of the cylindrical shell area is formed by a non-adhesive material relative to the upper surface of the sealing film, for example by metal, aluminium, stainless steel, Teflon, silicone, and the like, which does not exert a sufficiently strong interaction/bond with the upper surface of the sealing film under pressure on the sealing film. The cut-out surface of the shell may be formed of a ferromagnetic material, for example steel, when a magnetic clamping element is used.

The sealing film can be attached to the sealer in a variety of ways, preferably, the sealing film sheet has at the both ends thereof separately formed edge strips or wings, also provided with a covering layer strip, which do not need to be removed manually and can be used to attach the sealing film to the sealer body by means of clamping elements, such as clips, magnets, and the like. It is also possible to produce sealing film sheets with wings along the longer side of the sheet and a shape of the sealer corresponding thereto, but this is less advantageous since standard commercially produced sheets would not be used.

### Description of the Drawings

The subject of the invention is further illustrated by the following exemplary embodiments thereof, which are described with reference to the accompanying drawings, in which:
Fig. 1 is a schematic illustration of a sealer according to the invention with a semi-cylindrical body and with a clamping element in the form of a housing with magnets and barriers
Fig. 2 is a schematic illustration of a sealer according to the invention with a clamping element in the form of a clip
Fig. 3 is a schematic illustration of a sealer according to the invention with a clamping element in the form of simple magnets
Fig. 4 shows the attachment of the sealing film
Fig. 5 is a schematic illustration of a sealer body with a cut-out surface made as a straight line between the end points of a missing part of a circle
Fig. 6 is a schematic illustration of a sealer body with a cut-out surface made as a typical 90° circular sector
Fig. 7 is a schematic illustration of a sealer body with a cut-out surface made as a typical 270° circular sector
Fig. 8 is a schematic view of a detail of an expanded clamping element in the form of a housing with barriers
Fig. 9 is a schematic view of a detail of a compressed clamping element in the form of a housing with barriers

### Exemplary Embodiments of the Invention

Said embodiments illustrate exemplary variants of the embodiment of the invention, which, however, have no limiting effect on the scope of protection.

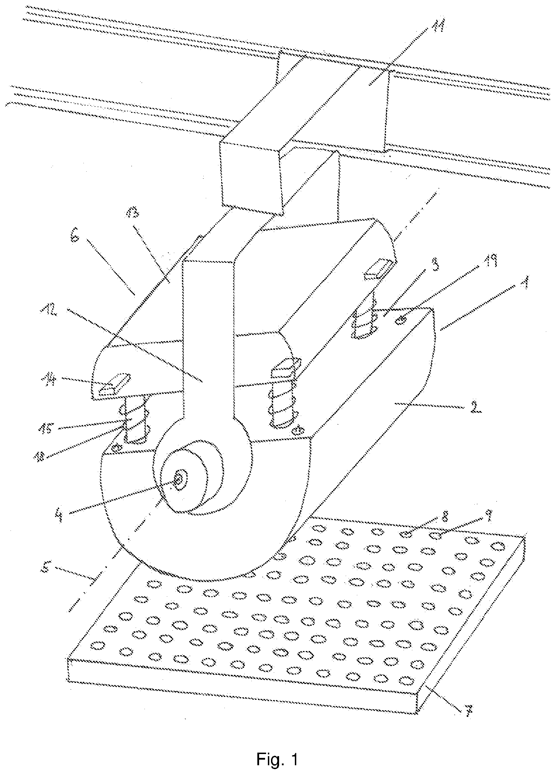

One example of an embodiment of the invention is a sealer for gas-tight sealing of wells 8 in PCR plates 7 by means of a sealing film shown in Fig. 1, having a sealer body 1 in the form of a partially cut-out cylinder, namely a semi-cylinder, i.e. with a segment of a circular base with an angle of 180°. The sealer further comprises a hinge 12 and a clamping element 6 in the form of a housing 13 with four magnets 14 and four barriers 15 for attaching the sealing film ends to the sealer body 1. Furthermore, the sealer in Fig. 1 has a hollow sealer body 1, which is partially formed by a cylindrical shell area 2 of the housing for forcefully transferring the sealing film to the upper rigid edge portion of the wells by pressure and swaying-rolling motion of the sealer body, a cut-out shell area 3 of rectangular shape and side plates. Side panels of the sealer body 1 are semi-circular panels connected to a hinge 12 by a rotary joint 4. The rotation axis 5 of the sealer body 1 is identical to the axis of an imaginary cylinder, which is based on a cylindrical shell area 2. Further, the hinge 12 is slidably coupled to a programmable positioning device 11. In another exemplary embodiment, entire side panels of the sealer body 1 can spin within the peripheral walls of the cylinder and thus be part of the rotary joint 4.

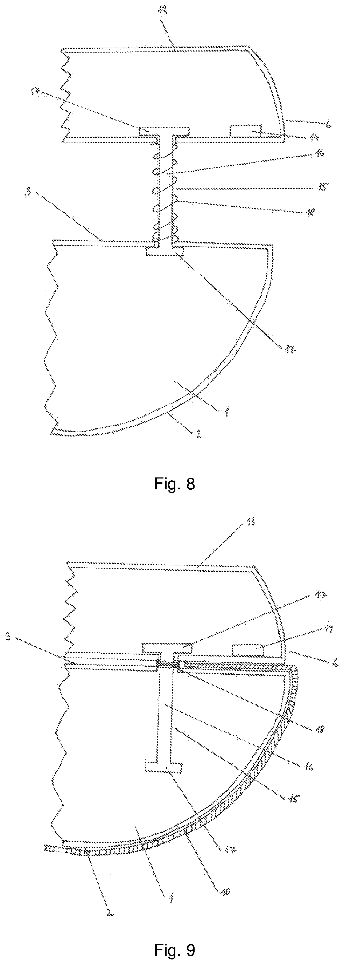

In the exemplary embodiment of Fig. 1, the clamping element 6 is in the form of a housing 13, where the lower part of the housing 13 copies the rectangular cut-out shell area 3 dimensionally and shape-wise and four magnets 14 are arranged in the lower portion of the housing 13. The cut-out shell area 3 of the housing is made of steel. The cylindrical shell area 2 is made of silicone. The housing 14 is connected to the cut-out shell area 3 by means of four barriers 15 consisting of the barrier body 16 and two heads 17, where one barrier 15 head 17 is housed in the hollow sealer body 1 and the second barrier head 17 is housed in the hollow housing 13, which is shown in detail in Figs. 8 and 9. The barrier body 16 extends through an opening in the cut-out surface 3 of the housing and through an opening in the lower portion of the housing 13, where each opening is narrower than the respective head 17. The body 16 of each barrier is provided with a spreader spring 18 that overcomes the magnetic force of the sealer body 1 separated from the housing 13 but does not overcome the magnetic force of the housing 13 pressed to the sealer body 1. Magnets 14 and barriers 15 are distributed evenly at the corners of the rectangle at the inner circumference of the cut-out shell area 3. The barrier 15 is formed by a metal rod with threads in the end portions and a metal nut at each end of the rod. In another exemplary embodiment, a bolt with a large head and a nut at the other end are used as a barrier 15. In this exemplary embodiment, the housing 13 is made of plastic, and in other exemplary embodiments made of Teflon, aluminium, stainless steel, and silicone were used. In other exemplary embodiments, the cylindrical shell area is made of metal, aluminium, stainless steel, Teflon.

Thus, if the housing 13 is separated from the sealer body 1, manually or accidentally, after releasing the sealing film, it is kept separate by means of springs 18 until it is manually pressed against the sealer body 1. After compression, the housing 13 retains by the sealer body 1 by virtue of the greater magnetic force, while at the same time, if the sealing film 10 is beforehand clamped between them, it holds the sealing film 10 by the ends thereof, until the sealing film 10 is transferred and pressed to the PCR plate 7, where the forces between the sealing film 10 and the PCR plate 7 prevail, and by moving the sealer, the ends of the sealing film 10 initially fixed by the magnetic force between the clamping element 6 and the sealer body 1 are drawn out.

The sealer according to Fig. 1 comprises four resilient pins 19 with a tip for securely attaching the sealing film 10, which conduct electric current when no sealing film is inserted between the sealer body and the pressure element. These pins 19 are attached to a spring latch. In the absence of the sealing film 10, sufficient pressure is not exerted on them and no sufficient pressure is recorded; they are in rest position. In the presence of the sealing film 10 pressed against the sealer body 1, the pins 19 with tips are more pressed into the sealer body 1 relative to the rest position, and no current passes through them, which is recorded by the analyser for automatic diagnostic PCR process. Pins 19 with tips are located near the edges of the end portions of the inserted attached sealing film 10 and, upon detecting a poor sealing film 10 position, the automatic diagnostic PCR process analyser detects and signals this error using pins 19 with tips prior to initiating the analytical diagnostic PCR process.

The cylindrical surface 2 of the housing in the exemplary embodiment of Fig. 1 corresponds to the dimensions of the typical analytical PCR plate 7, i.e. it has dimensions of 115 mm by 80 mm, which correspond to the commercially produced sealing film 10 for PCR analysis, so that the edges of the sealing film 10 are folded under the clamping element 6, wherein the sealing film 10 has a total dimension of 140 mm x 80 mm and the dimension without folded edges occupies 115 mm x 80 mm, and the dimensions of the cylindrical surface 2 are adapted to this dimension.

In another exemplary embodiment, the cylindrical surface 2 has dimensions of 125 mm by 85 mm.

In further exemplary embodiment, the cylindrical surface 2 has dimensions of 80 mm by 60 mm.

Detail of said clamping element 6 of Fig. 1 in the expanded state is shown in Fig. 8 and in the compressed state in Fig. 9.

The sealer was designed to seal wells 8 in PCR plates 7 filled, at least partly, with chemicals for PCR analysis. Before initiating the PCR analysis, the clamping element 6 was first separated from the sealer body 1, a protective cover was removed from the centre of the commercially-produced sealing film 10 for PCR analysis, and, in an open analyser for automatic diagnostic process, the sealing film 10 was manually attached to the cylindrical shell area 2 of the semi-cylindrical sealer body 1 so that the central portion of the film 10 was applied on the cylindrical surface shell 2, and each end provided with a strip of protective cover of rectangular sheet of the sealing film 10 was folded to the cut-out shell area 3 and these ends were attached by pressing the clamping element 6 in the form of a housing 13 with magnets 14. In embodiments of isolating nucleic acids and other preparatory operations and analyser settings (PCR Setup) for automatic diagnostic PCR process, liquid chemicals for PCR analysis were automatically pipetted into PCR plate 7. In the next step, the PCR plate 7 had to be gas-tight sealed, which was done by automated programmed steps in the analyser for automatic diagnostic PCR process, so that the sealer provided with the sealing film 10 was brought into position above the PCR plate 7 for gas-tight sealing. Furthermore, the sealing film 10 was pressed against the PCR plate 7 by means of the programmed positioning device 11 and the sealer, in particular onto the upper rigid edge portion 9 of the wells 8 and substantially onto the entire upper surface of the PCR plate 7, and transferred onto the upper surface of the PCR plate 7 by means of triple forward and reverse swaying-rolling motion, substantially by rolling from one end of the PCR plate to the other, wherein the ends of the sealing film 10 were spontaneously drawn out of the clamp between the cut-out shell area 3 and the clamping element 6. Thereby, all wells 8 in the PCR plate 7 were gas-tight sealed. Subsequently, the sealer was put into the parking position so as not to interfere with the following actual PCR analysis and the PCR plate 7 sealed by means of the sealing film 10 was further covered and pressed by a heating plate, and actual PCR analysis was performed in an automated manner using polymerase chain reaction, amplification and detection of the target nucleic acid molecule.

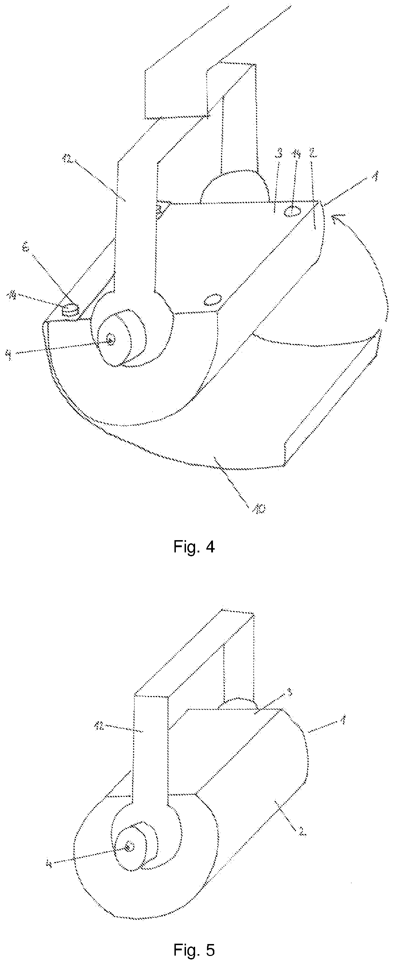

Fig. 4 illustrates a method of inserting and attaching a sealing film 10 to a sealer body 1, wherein a rectangular sealing film 10 comprises two ends with separate protective cover strips, the so-called wings, which are inserted between the clamping element 6 and the cut-out shell area 3 of the sealer body 1, along with the protective cover not being removed.

In another exemplary embodiment, the PCR plate in a separate rack was sealed with a sealer according to the invention.

A further exemplary embodiment is a not shown embodiment of the clamping element 6 in the form of a housing 13 with one magnet 14 and one barrier 15.

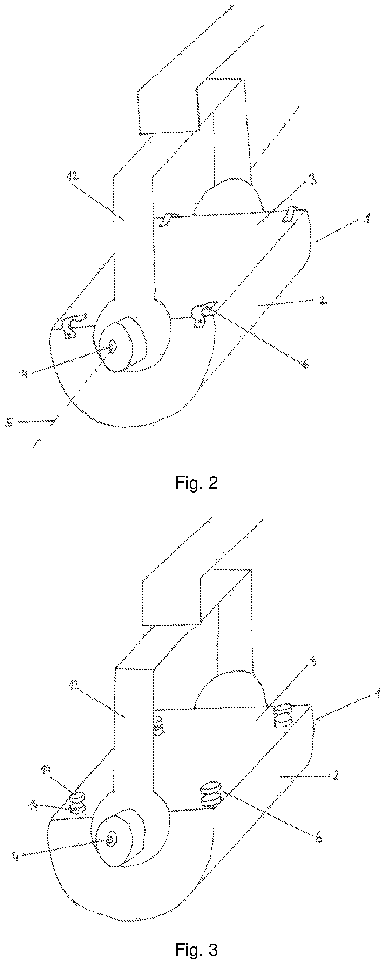

The clamping element 6 may be of various other embodiments ensuring that the sealing film 10 is held on the sealer body 1. These may be mutually attracting magnets 14, where one piece is implemented into the sealer body 1 and the other is adjacent to it through the sealing film 10, or magnets 14 located in the housing 13, wherein one of the magnetic counterparts may be replaced by a ferromagnetic material, as well as different clips, pins and other fasteners. Clamping elements 6 in the form of clips are shown in Fig. 2 or in the form of free magnets 14 as shown in Fig. 3.

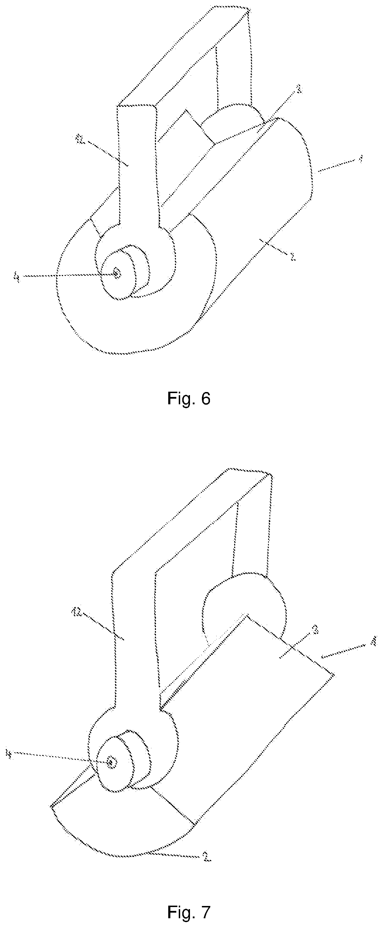

In the exemplary embodiments shown in Figs. 1 to 4, the sealer body 1 was semi-cylindrical, thus the cut-out shell area 3 was straight and relative to the side walls of the sealer body 1 constructed as a typical 180° circular sector. Exemplary embodiments of the sealer body 1 in a shape other than semi-cylindrical are shown, for example, in Figs. 5 to 7. Fig. 5 is a schematic illustration of a sealer body 1 with a straight cut-out surface 3 provided with respect to the side walls of the sealer body 1 as a straight line between the end points of the missing part of the circle. Fig. 6 is a schematic illustration of a sealer body 1 with a cut-out surface 3 formed by two mutually perpendicular planes and provided with respect to the side walls of the sealer body 1 as a typical 90° circular sector, and as a typical 270° circular sector in Fig. 7.

### Industrial Applicability

The sealer according to the invention is especially useful in laboratory or mobile equipment for PCR analyses. It is also possible to carry out the sealing of the sealing film on the PCR plate outside the analyser for automatic diagnostic PCR process in a separate rack. Another application relates to analyses employing analytical plates, such as microtiter plates, where it is necessary or appropriate to seal chemicals in analytical plates for various reasons, most often due to undesirable evaporation of chemicals.

### List of Reference Signs

- 1: - Sealer body
- 2: - Cylindrical shell area
- 3: - Cut-out shell area
- 4: - Rotary joint
- 5: - Rotation axis
- 6: - Clamping element
- 7: - PCR plate
- 8: - Well
- 9: - Upper rigid edge portion of wells
- 10: - Sealing film
- 11: - Positioning device
- 12: - Hinge
- 13: - Housing
- 14: - Magnet
- 15: - Barrier
- 16: - Barrier body
- 17: - Head
- 18: - Spring
- 19: - Pin

## Claims

1. A sealer for gas-tight sealing of wells in a PCR plate using a sealing film, whereas the sealer comprises a hinge (12) and further a sealer body (1) in the form of a longitudinally cut-out cylinder comprising a cylindrical shell area (2) for forcefully transferring the sealing film (10) to the upper rigid edge part (9) of the PCR plate (7) wells by pressure and swaying-rolling motion of the sealer body (1), and a cut-out shell area (3) where the sealer body (1) is rotatably connected to a hinge (12) by means of a joint (4) with a rotation axis coincident with a central axis (5) of the cylindrical surface the sealer further comprises at least one clamping element (6) for clamping both ends of the sealing film (10) to the sealer body (1), **characterised in that** the hinge (12) is further connected to a positioning device (11) for horizontal and/or vertical movement of the sealer in a plane perpendicular to the axis of rotation of the sealer body (1); the clamping element (6) is in the form of a housing (13), where the lower portion of the housing (6) dimensionally and shape-wise corresponds to the cut-out shell area (3) made of ferromagnetic material and at least one magnet (14) is located inside the lower portion of the housing (13), and the housing (13) is further connected to the cut-out shell area (3) by means of a barrier (15) consisting of a barrier body (16) provided at the ends thereof with two heads (17), where one barrier head (17) is housed in the hollow body of the sealer (1) and the second barrier head (17) is housed in the housing (13), and the barrier body (16) extends through an opening in the cut-out shell area (3) and an opening in the lower housing (13) part, where each opening is smaller than the respective head (17), and the barrier body (16) is provided with a spreader spring (18) that overcomes the magnetic force of the sealer body (1) separated from the housing (13) but does not overcome the magnetic force of the housing (13) pressed to the sealer body (1).

2. The sealer according to claim 1, **characterised in that** the cylindrical shell area (2) occupies a cylinder shell in the range of an angle of 90° to 270° of the circular base, and the remaining portion up to 360° is formed by the cut-out shell area (3), wherein the cylindrical shell area (2) is a rectangle suitable for covering at least the area defined by the wells (8) and by the upper rigid edge portion (9) thereof on the PCR plate (7) for sealing, preferably suitable for dimensions of the PCR plate of 115 mm by 80 mm.

3. The sealer according to claim 1 or 2, **characterised in that** the sealer body (1) has a semi-cylindrical shape.

4. The sealer according to any one of claims 1 to 3 , **characterised in that** four magnets (14) are arranged in the housing (13) in the vicinity of the corners of the rectangular lower part of the housing (13) and the housing (13) is connected with the cut-out shell area (3) by four barriers provided with springs.

5. The sealer according to any one of claims 1 to 4, **characterised in that** the cut-out shell area (3) of the sealer body (1) shell comprises at least one, preferably four resilient pins (19) made of electrical current conducting material and connected to the power supply to detect the location of the sealing film, where each pin (19) is provided with a tip to prevent the movement of the attached sealing film (10).

## Patentansprüche

1. Ein Abdichtungsmittel zum gasdichten Abdichten von Vertiefungen in einer PCR-Platte unter Verwendung einer Abdichtungsfolie, wobei das Abdichtungsmittel ein Scharnier (12) und ferner einen Abdichtungskörper (1) in Form eines in Längsrichtung ausgeschnittenen Zylinders umfasst, der einen zylindrischen Mantelbereich (2), zur kraftschlüssigen Übertragung der Abdichtungsfolie (10) auf den oberen starren Randabschnitt (9) der Vertiefungen von PCR-Platte (7) durch Druck-und Schwingen-Rollenbewegung des Abdichtungskörpers (1), und einen ausgeschnittenen Mantelbereich (3) umfasst, wobei der Abdichtungskörper (1) mit dem Scharnier (12), mittels eines Gelenks (4) mit einer mit einer Mittelachse (5) der zylindrischen Oberfläche zusammenfallenden Drehachse, drehbar verbunden ist, wobei das Abdichtungsmittel ferner mindestens ein Klemmelement (6), zum Festklemmen beider Enden der Abdichtungsfolie (10) auf dem Abdichtungskörper (1), umfasst, **dadurch gekennzeichnet, dass** das Scharnier (12) ferner mit einer Positioniereinrichtung (11), zur horizontalen und/oder vertikalen Bewegung des Abdichtungsmittels in einer Ebene senkrecht zur Drehachse des Abdichtungskörpers (1), verbunden ist; wobei das Klemmelement (6) in Form eines Gehäuses (13) ausgebildet ist, wobei der untere Abschnitt des Gehäuses (13) dem ausgeschnittenen Mantelbereich (3) aus ferromagnetischem Material größenmäßig und formmäßig entspricht und mindestens ein Magnet (14) innerhalb des unteren Abschnitts des Gehäuses (13) angeordnet ist, und das Gehäuse (13) ferner über eine Sperre (15) mit dem ausgeschnittenen Mantelbereich (3) verbunden ist, wobei die Sperre (15) aus einem Sperrkörper (16) besteht, der an seinen Enden mit zwei Köpfen (17) versehen ist, wobei ein Sperrkopf (17) in dem Hohlkörper des Abdichtungsmittels (1) untergebracht ist und der zweite Sperrkopf (17) in dem Gehäuse (13) untergebracht ist, und der Sperrkörper (16) sich durch eine Öffnung im ausgeschnittenen Mantelbereich (3) und eine Öffnung im unteren Abschnitt des Gehäuses (13) erstreckt, wobei jede Öffnung kleiner ist als der jeweilige Kopf (17), und der Sperrkörper (16) mit einer Spreizfeder (18) versehen ist, die Magnetkraft des vom Gehäuse (13) getrennten Abdichtungskörpers (1) überwindet, aber die Magnetkraft des an den Abdichtungskörper (1) angepressten Gehäuses (13) nicht überwindet.

2. Das Abdichtungsmittel nach Anspruch 1, **dadurch gekennzeichnet, dass** der zylindrische Mantelbereich (2) einen Zylindermantel, im Bereich eines Winkels von 90° bis 270° der kreisförmigen Basis, einnimmt und der verbleibende Abschnitt bis zu 360° durch den ausgeschnittenen Mantelbereich (3) gebildet ist, wobei der zylindrische Mantelbereich (2) ein Rechteck ist, das geeignet ist, zumindest den von den Vertiefungen (8) und von den oberen starren Randabschnitt (9) davon definierten Bereich auf der PCR-Platte (7) zur Abdichtung zu bedecken, vorzugsweise geeignet für Abmessungen der PCR-Platte von 115 mm mal 80 mm.

3. Das Abdichtungsmittel nach Anspruch 1 oder 2, **dadurch gekennzeichnet, dass** der Abdichtungskörper (1) eine halbzylindrische Form aufweist.

4. Das Abdichtungsmittel nach einem der Ansprüche 1 bis 3, **dadurch gekennzeichnet, dass** im Gehäuse (13) in der Nähe der Ecken des rechteckigen unteren Abschnitts des Gehäuses (13) vier Magnete (14) angeordnet sind und das Gehäuse (13) mit dem ausgeschnittenen Mantelbereich (3) durch vier mit Federn versehene Sperren verbunden ist.

5. Das Abdichtungsmittel nach einem der Ansprüche 1 bis 4, **dadurch gekennzeichnet, dass** der ausgeschnittene Mantelbereich (3) des Abdichtungskörpers (1) mindestens einen, vorzugsweise vier federnde Stifte (19) umfasst, der/die aus elektrisch stromleitendem Material Ist/sind und mit der Stromversorgung verbunden ist/sind, um den Ort der Abdichtungsfolie zu erfassen, wobei jeder Stift (19) mit einer Spitze versehen ist, um die Bewegung der angebrachten Abdichtungsfolie (10) zu verhindern.

## Revendications

1. Un dispositif de scellement étanche aux gaz de puits dans une plaque de PCR utilisant un film de scellement, où le dispositif de scellement comprend une charnière (12) et en outre un corps de scellement (1) sous la forme d'un cylindre découpé longitudinalement comprenant une zone de coque cylindrique (2), pour transférer de force le film de scellement (10) à la partie de bord rigide supérieure (9) des puits de la plaque de PCR (7) par pression et par un mouvement de roulement oscillant du corps de scellement (1), et une zone de coque découpée (3), où le corps de scellement (1) est relié de manière rotative à la charnière (12) à l'aide d'un joint (4) ayant un axe de rotation coïncidant avec un axe central (5) de la surface cylindrique, où le dispositif de scellement comprend en outre au moins un élément de serrage (6) pour serrer les deux extrémités du film de scellement (10) sur le corps de scellement (1), **caractérisé en ce que** la charnière (12) est en outre reliée à un dispositif de positionnement (11) pour un mouvement horizontal et/ou vertical du dispositif de scellement dans un plan perpendiculaire à l'axe de rotation du corps de scellement (1); l'élément de serrage (6) se présente sous la forme d'un boîtier (13), où la partie inférieure du boîtier (13) corresponde en dimensions et en forme à la zone de coque découpée (3) en matériau ferromagnétique et au moins un aimant (14) est situé à l'intérieur de la partie inférieure du boîtier (13), et le boîtier (13) est en outre relié à la zone de coque découpée (3) au moyen d'une barrière (15) constituée d'un corps de barrière (16) pourvu à ses extrémités de deux têtes (17), où une tête de barrière (17) est logée dans le corps creux du dispositif de scellement (1) et la seconde tête de barrière (17) est logée dans le boîtier (13), et le corps de barrière (16) s'étend à travers une ouverture dans la zone de coque découpée (3) et une ouverture dans la partie inférieure du boîtier (13), où chaque ouverture est plus petite que la tête respective (17), et le corps de barrière (16) est pourvu d'un ressort d'étalement (18) qui surmonte la force magnétique du corps de scellement (1) séparé du boîtier (13) mais ne surmonte pas la force magnétique du boîtier (13) pressé sur le corps de scellement (1).

2. Le dispositif de scellement selon la revendication 1, **caractérisé en ce que** la zone de coque cylindrique (2) occupe une enveloppe de cylindre dans la plage d'un angle de 90° à 270° de la base circulaire, et la partie restante jusqu'à 360° est formée par la zone de coque découpée (3), où la zone de coque cylindrique (2) est un rectangle apte à recouvrir au moins la zone définie par les puits (8) et par la partie de bord rigide supérieure (9) de ceux-ci sur la plaque de PCR (7) pour la sceller, de préférence appropriée pour des dimensions de la plaque de PCR de 115 mm de 80 mm.

3. Le dispositif de scellement selon la revendication 1 ou 2, **caractérisé en ce que** le corps de scellement (1) a une forme semi-cylindrique.

4. Le dispositif de scellement selon l'une quelconque des revendications 1 à 3, **caractérisé en ce que** quatre aimants (14) sont disposés dans le boîtier (13) au voisinage des coins de la partie rectangulaire inférieure du boîtier (13) et le boîtier (13) est relié à la zone de coque découpée (3) par quatre barrières pourvues de ressorts.

5. Le dispositif de scellement selon l'une quelconque des revendications 1 à 4, **caractérisé en ce que** la zone de coque découpée (3) de la coque du corps de scellement (1) comprend au moins une, de préférence quatre broches élastiques (19) constituées d'un matériau conducteur de courant électrique et connectées à l'alimentation électrique pour détecter l'emplacement du film de scellement, où chaque broche (19) est pourvue d'une pointe pour empêcher le mouvement du film de scellement (10) fixé.
